# EUROPEAN PATENT SPECIFICATION

(11) **EP 1 016 427 B1**
(45) Date of publication and mention of the grant of the patent: **03.11.2004**
(21) Application number: 99403280.3
(22) Date of filing: 24.12.1999
(51) Int. Cl.: A61M 5/178, A61M 5/145

(54) **A method and device for filing a syringe with a chemical solution**
Methode und Vorrichtung zum Befüllen einer Spritze mit einer chemischen Lösung
Méthode et dispositif pour remplir une seringue avec une solution chimique

(30) Priority: 28.12.1998 JP 37309598
(43) Date of publication of application: 05.07.2000
(73) Proprietor: Nemoto Kyorindo Co., Ltd., Tokyo (JP)
(72) Inventor: Nemoto, Shigeru, Bunkyo-ky, Tokyo (JP)
(74) Representative: Boulinguiez, Didier

(56) References cited:
- EP-A- 0 293 958
- EP-A- 0 595 474
- EP-A- 0 619 122
- EP-A- 0 654 279
- US-A- 3 701 345
- US-A- 3 935 883
- US-A- 4 465 473
- US-A- 4 685 903

## Description

### Technical Field

The present invention relates to a device and a method for transferring a chemical solution (such as contrast agent used for magnetic resonance imaging, X-ray CT, angiography, urography and the like) from a container (such as a bottle) to a syringe so as to fill the syringe with the chemical solution.

### Background of the Invention

Contrast agents, such as those used for magnetic resonance imaging ("MRI") and X-ray CT imaging, are generally used in liquid form having a high viscosity. These contrast agents are generally sold and provided to hospitals or other testing facilities in a bottle or syringe. When the contrast agent is provided in a syringe, it can be directly attached to an automatic injector designed for the contrast agent and can be used at once. In this way, filled syringes allow for easy and simple administration to the patient. However, if the contrast agent is not supplied in syringe form, the agent must be transferred from the bottle to syringe prior to the administration to the patient.

Unfortunately, it takes a fair amount of time and labor to aspirate a viscous chemical solution by hand in order to transfer it to the syringe. Though it is possible to aspirate the contrast agent into a syringe using an automatic injector, this does cause some difficulties. For instance, the loading of a syringe is not possible during the injection of the contrast agent into patient. Also, it is very difficult to transfer the correct and precise volume necessary for the administration.

In addition, the volume of the contrast agent used during a procedure depends upon the patient's weight or the diagnostic region. In prior art techniques of using an automatic injector to fill a syringe, all or most of the chemical solution in the bottle was transferred to the syringe. The necessary volume was then set by adjusting the automatic injector to administer only the required amount, which inevitably left residual amounts of chemical solution in the syringe after administration. The contrast agent remaining in the syringe is unfortunately wasted.

US-A-3 701 345 shows an injector control system having the features of the preamble of claim 1.

### Summary of the Invention

The present invention overcomes the problems of the prior art technology. The objective of the present invention is to provide a filling device and a filling method which are used for slowly filling the syringe with correct and necessary volume of a viscous chemical solution. This filing device and method thus transfers the contrast agent from the container to syringe while minimizes the excess waste of the contrast agent remaining in the syringe after the procedure.

Accordingly, the present invention is directed to a filling device for filling a syringe with a chemical solution by transferring the chemical solution stored in a container to the syringe, comprising: a cylinder holder to hold the syringe barrel, a piston holder to hold the syringe piston, a driving mechanism to move the piston holder relatively to the cylinder holder, an input mechanism to input test conditions, and a calculation mechanism to calculate the volume of the chemical solution based on the test conditions, whereby the piston holder is allowed to move according to the volume calculated with the calculation mechanism.

Another aspect of the present invention is directed to a method for filling a syringe with a chemical solution by transferring the chemical solution stored in a container to the syringe, comprising the steps of: mounting a syringe barrel and a piston to a cylinder holder and a piston holder, respectively, connecting the tip of the syringe to the connecting tube attached to the container storing the chemical solution, inputting test conditions with an input mechanism in the filling device, calculating the necessary volume of the chemical solution for administration with a calculating means in the filling device, moving the piston holder based on the calculated chemical solution volume, thereby the necessary volume of the chemical solution is aspirated from the container and filled in the syringe.

In one embodiment of the present invention, the filling device may be deigned for exclusive use as the filling device. In another embodiment of the present invention, the filling device may also serve as an injector for injecting the chemical solution to the patient.

### Brief Description of the Drawings

Fig. 1 shows an embodiment of chemical solution filling device of this invention.
Fig. 2 is a flow chart showing the chemical solution filling method of this invention.
Fig. 3 is an enlarged view (perspective view) to show an example of chemical solution filling device of this invention.
Fig. 4 is an enlarged view (top view) to show an example of chemical solution filling device of this invention.
Fig. 5 shows an embodiment of chemical solution filling device of this invention.

### Detailed Description of the Invention

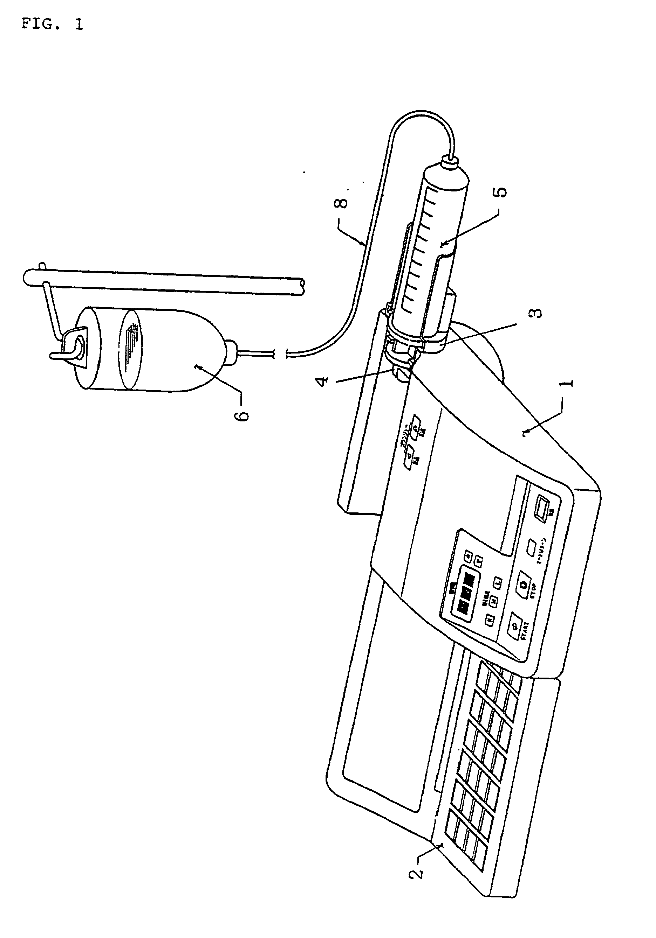

Figure 1 shows an embodiment of a chemical solution filling device of the present invention. The filling device shown in this embodiment comprises a main body 1 and a calculator portion 2. Equipped with the main body 1 are a cylinder holder 3 to a hold the barrel of a syringe 5, a piston holder 4 which holds the piston of the syringe 5 and can move relatively to the cylinder holder, and a driving portion, not shown in this figure, which includes a motor, also not shown, to move the piston holder 4. The calculation portion 2 contains an input interface such as a keyboard to input diagnostic test conditions, a display and a computer or other processing unit as a calculation and data storage means inside of the calculation portion 2.

As shown in Figure 1, the barrel and the piston of the syringe 5 are attached to the cylinder holder 3 and the piston holder 4, respectively. The syringe 5 is connected to the chemical solution bottle 6 through the connecting tube 8.

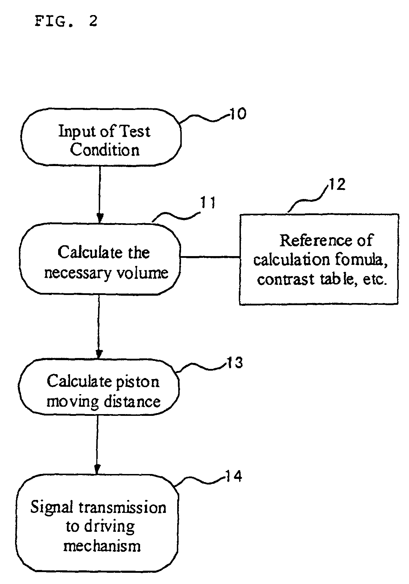

The method of using the present invention is shown in the flowchart in Figure 2. First the test conditions are input into the calculation portion 2 through the keyboard as shown in step 10. The test conditions used in a diagnostic X-ray CT, for instance, would include the scanning condition of X-ray CT, the test region, the patient's weight, and a description of the contrast agent. These actual input conditions should be sufficient to calculate the correct amount of contrast agent used for the procedure. The test conditions actually inputted in step 10 can be changed as necessary and appropriate for the particular test.

In step 11, the computer in the calculation portion 2 then calculates the volume of the contrast agent to be used. This calculation is based on the test conditions inputted in step 10. The necessary standard volume of contrast agent or calculation coefficient for each input item can be stored in database in advance and used to calculate the volume according to the test conditions. For example, the necessary standard injection volume to a patient with standard weight for each contrast agent is stored, and coefficients for standard injection volume on weight and scanning condition is prepared in numerical formula each in advance. This information is represented by box 12 in the flow chart of Figure 2. The correct and precise volume is then calculated based on this information as altered by the test conditions of step 10.

Once step 11 determines the injection volume of the chemical solution, the moving distance of the syringe piston is calculated in step 13. The moving distance is based upon the volume calculated in step 11 and the known cross-sectional area of the barrel of syringe 5. If syringes 5 of varying cross-sectional area are to be used in the present invention, then it is possible to determine which syringe 5 is being used by requiring that the type of syringe being used be inputted with the other test conditions in step 10. Alternately, a simple mechanical device (not shown in the figures) could be moved into contact with the syringe 5 to determine which of the possible syringes are being utilized. In addition to merely calculating the piston moving distance required to create the volume calculated in step 11, step 13 may also take into consideration the required moving distance of the piston to compensate for the dead space in the connecting tube 8.

Once the piston moving distance is determined, a signal is sent to the motor in the main body 1 to move the piston holder 4 (and hence the piston of syringe 5) the required distance in step 14. By so instructing the motor, the piston is moved and the required amount of chemical solution is aspirated into the syringe 5.

If the aspiration speed is too fast, the leakage of air from connectors or the damage of the syringe occurs frequently. Therefore it is also preferable that the aspirating condition of the filling device such as aspirating speed or aspirating pressure can be altered. Since the aspirating condition differs depending on the kind of the chemical solution and the type of the syringe and the like, it is specifically preferred that the aspirating condition should be calculated by the computer base on these data.

As mentioned previously, the filling device of the present invention may be deigned for exclusive use as the filling device or may also serve as an injector for injecting the chemical solution to the patient. In case the device is deigned for exclusive use as the filling device, the syringe filled with a chemical solution by this filling device will be set to an injector and the chemical solution is injected to a patient through delivery extension tube attached to the tip of the syringe. In case the device also serves as an injector, injection is conducted through a delivery extension tube attached to the tip of the syringe in place of the connecting tube 8. Or injection may also be conducted by changing the path using three-way valve from a connecting tube to a delivery extension tube. To the end of the delivery tube further attached are the suitable apparatus for injection such as a butterfly needle and the like or catheters depending on the test regions.

When the injection of the chemical solution is conducted, the path though syringe tip to the test region in the patient's body may constitute a dead space. This dead space may also preferably be taken into consideration on determining the moving distance of the piston.

Using the filling device of this invention, the optimum volume of the chemical solution is filled in the syringe 5 with no need to recalculate or control the volume when the chemical solution is injected to the patient. Therefore it allows the use of an injection device with relatively simple mechanism as a device for the administration of chemical solution to the patient. Also there is no waste of chemical solution since only the necessary volume of chemical solution is transferred from the bottle to the syringe 5. It is especially effective when expensive chemical solution is used or the chemical solution is stored in a bottle of large capacity.

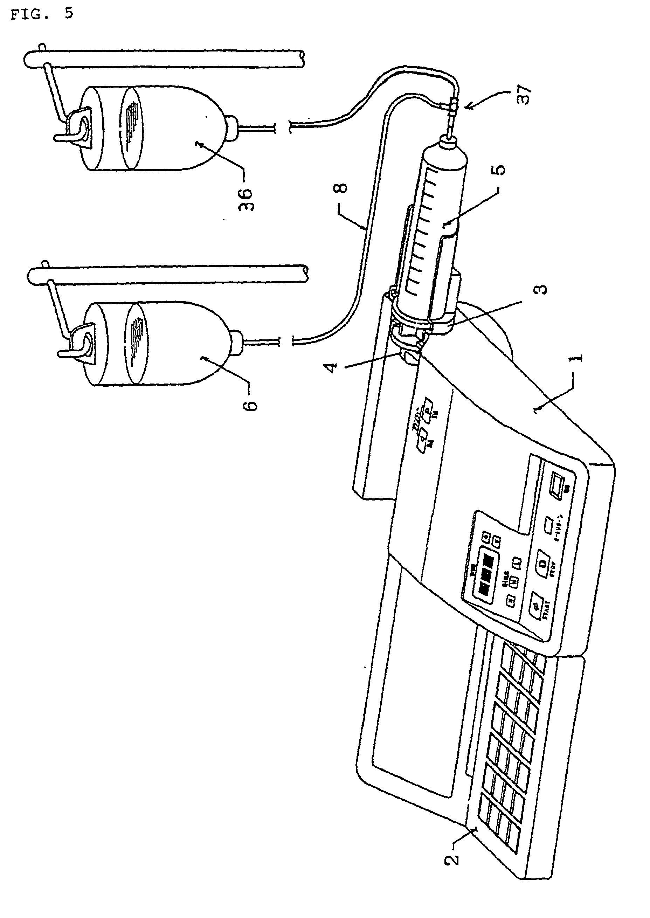

The embodiment explained so far is the case where only one kind of chemical solution is used. However, a chemical solution may be used after diluted with physiological saline solution or pure water to obtain suitable concentration of iodide, for example for X-ray CT, or suitable viscosity of the liquid. In such cases, the dilution ratio of the chemical solution is calculated by the computer to give the volume of the chemical solution and the diluent, thereby the moving distance of the piston is determined. The aspiration of the chemical solution and the diluent is achieved using the system shown in Fig. 5, for example. In this system the path of the chemical solution from chemical solution bottle 8 and the diluent bottle 36 may be switched using a three-way valve 37. The switching maybe operated manually or automatically synchronous to the movement of the piston.

The example of a contrast agent for X-ray CT was used above, although this device can be used to transfer other chemical solutions, both viscous and non-viscous. The main purpose is to transfer other viscous solutions, however, such as contrast agents for MRI, angiography or urography.

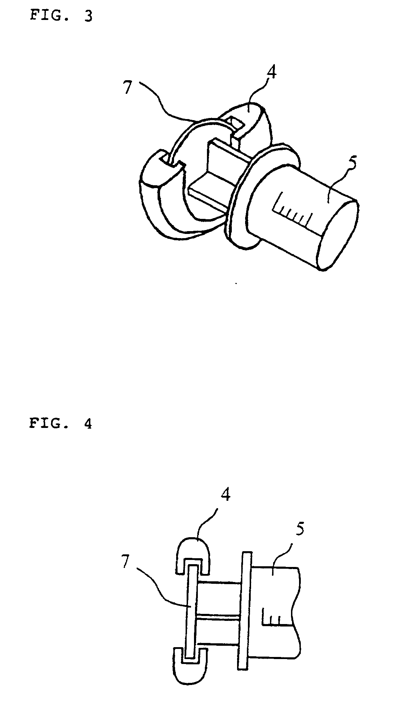

The only requirement for the cylinder holder 3 and the piston holder 4 is that they are adapted to the barrel and piston, respectively, of the syringe 5 being used. For example, Figure 1 shows cylinder holder 3 and piston holder 4 that fit closely with the brim of the syringe barrel and the syringe piston. Figures 3 and 4 show enlarged views of the brim of the piston 7 being held by the piston holder 4. In the preferred embodiment, the channel of the piston holder 4 is wider by about 1 mm than thickness of the piston brim 7 in order to make it easier to put on and take off the piston 5. Although the piston holder 4 is shown in the figures with a semi-circular shape having an interior groove slightly larger the thickness of a circular piston brim 7, nothing in the present invention is intended to limit the piston holder 4 to such shapes. For instance, those with standard skill in the art would be aware of numerous other configurations of piston brims 7, and could develop corresponding piston holders 4 to hold such brims 7. These other configurations are well within the scope of the present invention.

There is also no particular limitation for the mechanism used to move the piston holder 4 the distance calculated in step 13. For instance, the mechanism might utilize a stepping motor, a ball screw shaft and a ball screw nut that is engaged with the ball screw shaft and supports the piston holder 4. In this case, the stepping motor would drive the rotation of the ball screw shaft based upon the signal transmitted from the calculating mechanism as described in connection with step 14. This causes the movement of the ball screw nut together with the piston holder in the longitudinal direction of the ball screw shaft axis. The necessary interface to drive the stepping motor the calculated distance is well known in the prior art.

Although not described above in connection with Figure 2, it is also possible to include a preliminary step in the aspiration process to remove air from the syringe 5 and connecting tube 8. This step would involve programming the aspirator to aspirate the necessary volume of chemical solution after confirming that syringe piston is pushed automatically all the way to the end. This would result in all air being removed from the system prior to aspiration of the chemical solution.

Although the embodiment illustrated in Fig. 1 shows the example of single-piece construction in which the calculating portion 2 and the piston driving main body 1 are accommodated in one housing, separate-type construction can also be used. It is desirable that the motion of forward, backward and stop, and aspiration speed (moving speed) and the like can be set or controlled not only at the calculating portion 2, but also at the main body 1 of the aspiration device. Controls for doing so are shown on the main body 1 in Figure 2. This enables both manual operation and automatic operation. While these controls are present on the preferred embodiment shown in the figures, such controls are not necessary and it is possible that controls are located within the calculating portion 2.

In the embodiment shown in the figures, the input means of the calculating portion 2 is shown as a keyboard. However, it is within the scope of the present invention to use any other input interface known in the art. For instance, it may be preferred in some cases to transmit the data of the patient directly from the host computer of the hospital into the calculating portion 2. Alternatively, the interface of the calculating portion may be simple selection switches.

Although it is not the main objective of the present invention, the filling device of the present invention may be used as an injector for pre-filled type syringe. In this case, although a surplus of the chemical solution may be wasted, the injection can be conducted easily.

The invention is not to be taken as limited to all of the details set forth above, as modifications and variations to these details may be made without departing from the scope of the invention as defined in the claims.

## Claims

1. A filling device for filling a syringe (5) with a chemical solution stored in a container (6, 36), the syringe having a barrel and a piston, the filling device comprising:
a) a cylinder holder (3) adapted to hold the syringe barrel;
b) a piston holder (4) adapted to hold the syringe piston;
c) a driving mechanism attached to the piston holder, the driving mechanism being capable of moving the piston holder relative to the cylinder holder;
**characterized in that** the device further comprises
d) an input mechanism adapted to receive inputted test conditions; and
e) a calculation portion (2) to calculate an appropriate volume of the chemical solution based on the test conditions,
whereby the driving mechanism moves the piston holder according to the volume calculated by the calculation portion.

2. The filling device according to Claim 1, wherein the chemical solution comprises a contrast agent.

3. The filling device according to Claim 1, wherein the chemical solution is a contrast agent for X-ray CT, magnetic resonance imaging, angiography or urography.

4. The filling device according to Claim 1, wherein the device further comprises a data storage mechanism.

5. The filling device according to Claim 1, wherein the input mechanism is a keyboard.

6. The filling device according to Claim 1, wherein the calculation portion is a computer.

7. The filling device according to Claim 4, wherein the data storage mechanism is a data storage system in a computer.

8. The filling device according to Claim 1, wherein the driving mechanism comprises a stepping motor, a ball screw shaft, and a ball screw nut engaged with the ball screw shaft.

9. The filling device according to Claim 1, wherein the driving mechanism and the calculation mechanism are accommodated in one housing.

10. The filling device according to Claim 1, wherein the test conditions are selected from the group including scanning condition, test region, patient's weight, and contrast agent description.

11. The filling device according to Claim 1, wherein the test conditions include an indication of the cross-sectional area of the syringe.

12. A syringe filling system comprising:
a) a syringe (5) having a piston and a barrel of a known cross-sectional area; and
b) a filling device according to claim 1, wherein the calculation portion comprises a processing unit for calculating a volume in response to test conditions and a signal generator that generates a driving signal to instruct the driving mechanism to move the piston holder sufficiently so as to fill the syringe according to the calculated volume.

13. The syringe filling system of claim 12, wherein the input mechanism is a data receiving interface for receiving test conditions.

14. The syringe filling system of claim 13, further comprising a bottle containing a liquid, and a connecting tube (8) connecting the bottle to the syringe, wherein the driving signal compensates for the dead space in the connecting tube.

15. The syringe filling system of claim 13, further comprising a means for determining the cross-sectional area of the syringe.

16. The syringe filling system of claim 13, wherein the data receiving interface is a computer to computer interface for receiving data from a separate computer containing patient data.

17. The syringe filling system of claim 13, further comprising a data storage media containing standard usage information regarding the use of at least one contrast agent, wherein the processing unit uses the information in the data storage media along with the test conditions to calculate the volume.

18. The syringe filling system of claim 12 or 13, wherein the piston has a piston brim and the piston holder is adapted for receiving the piston brim.

19. A method for filling a syringe with a chemical solution by transferring the chemical solution stored in a container to the syringe, comprising the steps of:
a) mounting a syringe barrel and a piston to a cylinder holder and a piston holder, respectively, equipped in a filling device,
b) connecting the tip of the syringe to a connecting tube attached to the container storing the chemical solution,
c) inputting test conditions with an input means in the filling device,
d) calculating necessary volume of the chemical solution for administration with a calculating means in the filling device, and
e) moving the piston holder based on the calculated chemical solution volume, whereby the necessary volume of the chemical solution is aspirated from the container and filled in the syringe.

20. The method of claim 19, wherein the process of inputting the test conditions includes inputting an indication of the size of the syringe barrel.

21. The method of claim 19, further comprising the step of determining the cross-sectional area of the syringe.

22. The method of claim 19, further comprising the step of removing air from the syringe and connecting tube by aspirating a volume of chemical solution after the syringe piston is pushed automatically all the way to the end.

## Patentansprüche

1. Füllvorrichtung zum Befüllen einer Spritze (5) mit einer chemischen Lösung, die sich in einem Behälter (6, 36) befindet, wobei die Spritze einen Zylinder und einen Kolben hat, und die Füllvorrichtung folgendes aufweist:
a) einen zylindrischen Halter (3), welcher derart beschaffen und ausgelegt ist, daß er den Spritzenzylinder hält;
b) einen Kolbenhalter (4), welcher derart beschaffen und ausgelegt ist, daß er den Spritzenkolben hält;
c) eine Antriebseinrichtung, welche an dem Kolbenhalter angebracht ist, wobei die Antriebseinrichtung den Kolbenhalter relativ zum Zylinderhalter bewegen kann;
**dadurch gekennzeichnet, daß** die Vorrichtung ferner folgendes aufweist:
d) eine Eingabeeinrichtung, welche derart beschaffen und ausgelegt ist, daß sie eingegebene Testbedingungen aufnimmt; und
e) ein Ermittlungsteil (2) zur Ermittlung eines geeigneten Volumens der chemischen Lösung basierend auf den Testbedingungen,
wobei die Antriebseinrichtung den Kolbenhalter nach Maßgabe des durch das Ermittlungsteil ermittelten Volumens bewegt.

2. Füllvorrichtung nach Anspruch 1, bei der die chemische Lösung ein Kontrastmittel aufweist.

3. Füllvorrichtung nach Anspruch 1, bei der die chemische Lösung ein Kontrastmittel für Röntgenstrahl-Computertomographie, eine Kernspinnresonanzuntersuchung, eine Angiographie oder Urographie ist.

4. Füllvorrichtung nach Anspruch 1, bei der die Vorrichtung ferner eine Datenspeichereinrichtung aufweist.

5. Füllvorrichtung nach Anspruch 1, bei der die Eingabeeinrichtung eine Tastatur ist.

6. Füllvorrichtung nach Anspruch 1, bei der das Ermittlungsteil ein Computer ist.

7. Füllvorrichtung nach Anspruch 4, bei der die Datenspeichereinrichtung ein Datenspeicher in einem Computer ist.

8. Füllvorrichtung nach Anspruch 1, bei der die Antriebseinrichtung ein Schrittmotor, eine Kugelumlaufspindel und eine Kugelumlaufmutter aufweist, welche mit der Kugelumlaufspindel zusammenarbeitet.

9. Füllvorrichtung nach Anspruch 1, bei der die Antriebseinrichtung und die Ermittlungseinrichtung in einem Gehäuse untergebracht sind.

10. Füllvorrichtung nach Anspruch 1, bei der die Testbedingungen aus der Gruppe gewählt sind, welche Scannbedingungen, Testbereich, Gewicht eines Patienten und eine Beschreibung des Kontrastmittels umfaßt.

11. Füllvorrichtung nach Anspruch 1, bei der die Testbedingungen eine Angabe über die Querschnittsfläche der Spritze umfassen.

12. Spritzenfüllanlage, welche folgendes aufweist:
a) eine Spritze (5) mit einem Kolben und einem Zylinder mit einer bekannten Querschnittsfläche; und
b) eine Füllvorrichtung nach Anspruch 1, bei der das Ermittlungsteil eine Verarbeitungseinheit zum Ermitteln eines Volumens in Abhängigkeit von den Testbedingungen und einen Signalgenerator aufweist, welcher ein Antriebssignal erzeugt, um zu bewirken, daß die Antriebseinrichtung den Kolbenhalter in ausreichendem Maße derart bewegt, daß die Spritze nach Maßgabe des erforderlichen Volumens gefüllt wird.

13. Spritzenfüllanlage nach Anspruch 12, bei der die Eingabeeinrichtung eine Datenempfangsschnittstelle zum Empfangen der Testbedingungen ist.

14. Spritzenfüllanlage nach Anspruch 13, welche ferner eine eine Flüssigkeit enthaltende Flasche und eine Verbindungsleitung (8) aufweist, welche die Flasche mit der Spritze verbindet, wobei das Antriebssignal den Totraum in der Verbindungsleitung kompensiert.

15. Spritzenfüllanlage nach Anspruch 13, welche ferner eine Einrichtung zum Bestimmen der Querschnittsfläche der Spritze aufweist.

16. Spritzenfüllanlage nach Anspruch 13, bei der die Datenempfangsschnittstelle ein Computer ist, welcher eine Schnittstelle zum Empfangen von Daten von einem gesonderten Computer hat, welcher die Patientendaten enthält.

17. Spritzenfüllanlage nach Anspruch 13, welche ferner ein Datenspeichermedium aufweist, welches die Standardgebrauchsanweisungen bezüglich des Einsatzes von wenigstens einem Kontrastmittel enthält, wobei die Verarbeitungseinheit die Informationen in dem Datenspeichermedium zusammen mit den Testbedingungen zur Ermittlung des Volumens nutzt.

18. Spritzenfüllanlage nach Anspruch 12 oder 13, bei der der Kolben einen Kolbenrand hat, und der Kolbenhalter derart beschaffen und ausgelegt ist, daß er den Kolbenrand aufnimmt.

19. Verfahren zum Befüllen einer Spritze mit einer chemischen Lösung mittels Weiterleiten einer in einem Behälter enthaltenen chemischen Lösung zur der Spritze, welches die folgenden Schritte aufweist:
a) Anbringen eines Spritzenzylinders und eines Kolbens an einem Zylinderhalter und einem Kolbenhalter jeweils, welche in einer Füllvorrichtung vorgesehen sind,
b) Verbinden des vorderen Endes der Spritze mit einer Verbindungsleitung, welche an dem Behälter angebracht ist, welcher die chemische Lösung enthält,
c) Eingabe von Testbedingungen mittels einer Eingabeeinrichtung in die Füllvorrichtung,
d) Ermitteln des erforderlichen Volumens der chemischen Lösung zur Verabreichung mittels einer Ermittlungseinrichtung in der Füllvorrichtung, und
e) Bewegen des Kolbenhalters basierend auf dem ermittelten Volumen der chemischen Lösung, wobei das erforderliche Volumen der chemischen Lösung aus dem Behälter angesaugt und in die Spritze eingefüllt wird.

20. Verfahren nach Anspruch 19, bei dem das Verfahren zum Eingeben der Testbedingungen das Eingeben einer Angabe über die Abmessung des Spritzenzylinders umfaßt.

21. Verfahren nach Anspruch 19, welches ferner den Schritt aufweist, gemäß welchem die Querschnittsfläche der Spritze ermittelt wird.

22. Verfahren nach Anspruch 19, welches ferner den Schritt aufweist, gemäß welchem Luft aus der Spritze abgeführt wird, und die Leitung zum Ansaugen des Volumens der chemischen Lösung hiermit verbunden wird, nachdem der Spritzenkolben automatisch vollständig bis zum Hubende gedrückt worden ist.

## Revendications

1. Un dispositif de remplissage pour remplir une seringue (5) avec une solution chimique stockée dans un récipient (6, 36), la seringue ayant un fût et un piston, le dispositif de remplissage comprenant :
a) un support de cylindre (3) agencé pour retenir le fût de seringue ;
b) un support de piston (4) agencé pour retenir le piston de seringue ;
c) un mécanisme d'entraînement fixé au support de piston, le mécanisme d'entraînement étant capable de déplacer le support de piston par rapport au support de cylindre,
**caractérisé en ce que** le dispositif comprend, en outre :
d) un mécanisme d'entrée agencé pour recevoir les conditions d'essai introduites et
e) une partie de calcul (2) pour calculer un volume approprié de la solution chimique sur la base des conditions d'essai,
de sorte que le mécanisme d'entraînement déplace le support de piston selon le volume calculé par la partie de calcul.

2. Le dispositif de remplissage selon la revendication 1, dans lequel la solution chimique comprend un agent de contraste.

3. Le dispositif de remplissage selon la revendication 1, dans lequel la solution chimique est un agent de contraste pour la CT aux rayons X, l'imagerie par résonance magnétique, l'angiographie ou l'urographie.

4. Le dispositif de remplissage selon la revendication 1, dans lequel le dispositif comprend, en outre, un mécanisme de stockage de données.

5. Le dispositif de remplissage selon la revendication 1, dans lequel le mécanisme d'entrée est un clavier.

6. Le dispositif de remplissage selon la revendication 1, dans lequel la partie de calcul est un ordinateur.

7. Le dispositif de remplissage selon la revendication 4, dans lequel le mécanisme de stockage de données est un système de stockage de données dans un ordinateur.

8. Le dispositif de remplissage selon la revendication 1, dans lequel le mécanisme d'entraînement comprend un moteur pas à pas, un axe à vis à bille, et un écrou à vis à bille en prise avec l'axe à vis à bille.

9. Le dispositif de remplissage selon la revendication 1, dans lequel le mécanisme d'entraînement et le mécanisme de calcul sont reçus dans un boîtier.

10. Le dispositif de remplissage selon la revendication 1, dans lequel les conditions d'essai sont choisies parmi le groupe comprenant une condition d'exploration, une région d'essai, le poids du patient et la description d'un agent de contraste.

11. Le dispositif de remplissage selon la revendication 1, dans lequel les conditions d'essai comprennent une indication de la section transversale de la seringue.

12. Un système de remplissage de seringue comprenant :
a) une seringue (5) ayant un piston et un fût de section transversale connue et
b) un dispositif de remplissage selon la revendication 1, dans lequel la partie de calcul comprend une unité de traitement pour calculer un volume en réponse aux conditions d'essai et un générateur de signal qui génère un signal d'entraînement pour demander au mécanisme d'entraînement de déplacer le support de piston suffisamment afin de remplir la seringue selon le volume calculé.

13. Le système de remplissage de seringue selon la revendication 12, dans lequel le mécanisme d'entrée est une interface de réception de données pour recevoir les conditions d'essai.

14. Le système de remplissage de seringue selon la revendication 13, comprenant, en outre, une bouteille contenant un liquide et un tube de liaison (8) reliant la bouteille à la seringue, dans lequel le signal d'entraînement compense l'espace mort dans le tube de liaison.

15. Le système de remplissage de seringue selon la revendication 13, comprenant, en outre, un moyen pour déterminer la section transversale de la seringue.

16. Le système de remplissage de seringue selon la revendication 13, dans lequel l'interface de réception de données est une interface ordinateur à ordinateur pour recevoir les données à partir d'un ordinateur séparé contenant des données de patient.

17. Le système de remplissage de seringue selon la revendication 13, comprenant, en outre, des milieux de stockage de données contenant des informations d'utilisation classique concernant l'utilisation d'au moins un agent de contraste, dans lequel l'unité de traitement utilise l'information dans les milieux de stockage de données avec les conditions d'essai pour calculer le volume.

18. Le système de remplissage de seringue selon la revendication 12 ou 13, dans lequel le piston comprend un bord de piston et le support de piston est agencé pour recevoir le bord de piston.

19. Un procédé pour remplir une seringue avec une solution chimique en transférant la solution chimique emmagasinée dans un récipient à la seringue, comprenant les étapes de :
a) monter un fût de seringue et un piston dans un support de cylindre et un support de piston prévus respectivement dans un dispositif de remplissage,
b) relier l'extrémité de la seringue à un tube de liaison fixé au récipient stockant la solution chimique,
c) introduire des conditions d'essai avec un moyen d'entrée dans le dispositif de remplissage,
d) calculer le volume nécessaire de solution chimique pour l'administration avec un moyen de calcul dans le dispositif de remplissage et
e) déplacer le support de piston sur la base du volume de solution chimique calculé,
de sorte que le volume nécessaire de la solution chimique est aspiré du récipient et rempli dans la seringue.

20. Le procédé selon la revendication 19, dans lequel le procédé d'introduction des conditions d'essai comprend l'introduction d'une indication de la dimension du fût de seringue.

21. Le procédé selon la revendication 19, comprenant, en outre, l'étape de détermination de la section transversale de la seringue.

22. Le procédé selon la revendication 19, comprenant, en outre, l'étape d'élimination de l'air de la seringue et du tube de liaison en aspirant un volume de solution chimique après que le piston de seringue ait été poussé automatiquement sur tout le passage jusqu'à l'extrémité.
